# EUROPEAN PATENT SPECIFICATION

(11) **EP 4 461 719 B1**
(45) Date of publication and mention of the grant of the patent: **21.01.2026**
(21) Application number: 23210890.2
(22) Date of filing: 20.11.2023
(51) Int. Cl.: C07C 2/66, C07C 2/68, C07C 5/333, C07C 7/163, C07C 7/167, C07C 11/02, C07C 15/107, C10G 3/00, C10G 29/20, C10G 45/64, C10G 69/04, C10G 69/12

(54) **PROCESS FOR PRODUCING RENEWABLE MONO-METHYL ALKYLBENZENE PRODUCTS**
VERFAHREN ZUR HERSTELLUNG VON ERNEUERBAREN MONOMETHYLALKYLBENZOLPRODUKTEN
PROCÉDÉ DE PRODUCTION DE PRODUITS MONOMÉTHYLALKYLBENZÈNE RENOUVELABLES

(30) Priority: 30.05.2023 US 202363504881 P
(43) Date of publication of application: 13.11.2024
(73) Proprietor: UOP LLC, Rosemont, Illinois 60018 (US)
(72) Inventor: FICHTL, Geoffrey W., Charlotte, 28202 (US); DO, Phuong T.M., Charlotte, 28202 (US); WEXLER, James T., Charlotte, 28202 (US); DU, Hai, Charlotte, 28202 (US); JERORO, Eseoghene, Charlotte, 28202 (US); WHITCHURCH, Patrick C., Charlotte, 28202 (US)
(74) Representative: Boult Wade Tennant LLP

(56) References cited:
- US-A1- 2013 317 267
- US-A1- 2013 338 410
- US-A1- 2015 361 012

## Description

### BACKGROUND

Linear alkylbenzenes are organic compounds with the formula C₆H₅CₙH₂ₙ₊₁. While the alkyl carbon number, "n" can have any practical value, detergent manufacturers desire that alkylbenzenes have alkyl carbon number in the range of 9 to 16 and preferably in the range of 9 to 14. These specific ranges are often required when the alkylbenzenes are used as intermediates in the production of surfactants for detergents. The alkyl carbon number in the range of 9 to 14 falls in line with the specifications of the detergents industry.

Because the surfactants created from alkylbenzenes are biodegradable, the production of alkylbenzenes has grown rapidly since their initial uses in detergent production in the 1960s. The linearity of the paraffin chain in the alkylbenzenes is key to the material's biodegradability and effectiveness as a detergent. A major factor in the final linearity of the alkylbenzenes is the linearity of the paraffin component.

While detergents made utilizing alkylbenzene-based surfactants are biodegradable, previous processes for creating alkylbenzenes are not based on renewable sources. Specifically, alkylbenzenes are currently produced from kerosene refined from crude extracted from the earth. Due to the growing environmental prejudice against fossil fuel extraction and economic concerns over exhausting fossil fuel deposits, there may be support for using an alternate source for biodegradable surfactants in detergents and in other industries.

Some detergent manufacturers serve a specialty market based on alkylbenzenes enriched in mono-methyl alkyl benzenes (MMAB) relative to more typical linear alkyl benzenes (LAB).

Accordingly, it is desirable to provide MMAB that are made from biorenewable sources instead of being extracted from the earth. Further, it is desirable to provide renewable linear alkylbenzenes from vegetable, animal, nut, and/or seed oils to reduce the carbon intensity relative to fossil-based sources of mono-methyl paraffins.
US 2013/317267 A1 relates to methods for the co-production of alkylbenzene and biofuel from natural oils using hydrocracking.
US 2015/361012 A1 relates to methods for producing linear alkylbenzenes, paraffins, and olefins from natural oils and kerosene.
US 2013/338410 A1 relates to methods for producing linear alkylbenzenes from bio-renewable feedstocks.

### BRIEF DESCRIPTION OF THE DRAWINGS

Fig. 1 is a schematic view of one embodiment of a process for producing mono-methyl alkyl benzenes according to the present invention.
Fig. 2 is a plot of the mass-% normal paraffins versus deoxygenation temperature in accordance with Example 2.

### DETAILED DESCRIPTION

The invention is in accordance with the appended claims. The present invention relates to methods for producing a stream enriched in mono-methyl alkylbenzenes from. natural oils, such as vegetable, animal, nut, and/or seed oils, and triglyceride-containing oils. The method provides a stream enriched in mono-methyl paraffins relative to normal paraffins which can be used to produce the mono-methyl alkyl benzenes.

Normal paraffins are generated via deoxygenation and hydrogenation of triglyceride feeds, such as palm kernel oil (PKO), and other plant or animal based oils. The deoxygenation and hydrogenation takes place in the first step of the process by contacting the natural oil feed with a catalyst and hydrogen at elevated temperature and pressure. Some plant based oils, such as PKO or coconut, already have carbon chains that naturally fall in the C9 to C14 range typical in detergent applications. Longer chains can undergo selective hydrocracking to enrich the normal paraffin product of deoxygenation/hydrogenation in normal paraffins with carbon numbers of 9 to 14. From there, normal paraffins can be selectively lightly isomerized in a hydroisomerization step to create a portion of paraffins with mono-methyl branching. The mixture of paraffins can be further enriched in mono-methyl branched paraffins by processing through an adsorption separation system, and raffinate could be recycled to hydroisomerization to further make mono-methyl branched paraffins. The adsorption separation process for a large scale process may use a simulated moving bed design for continuous separation of components in the mixture. The simulated moving bed process is described in U.S. Pat. No. 2.985.589, for example. ZSM and X type zeolites have been used widely in absorption separation systems, as mentioned in US 6,225,518. Suitable adsorbents for the adsorbent system, include but are not limited to, a ZSM or a type X zeolite, such as ZSM-5 or 13X zeolites.

The stream enriched in mono-methyl paraffins is processed through contaminant removal, dehydrogenation, selective hydrogenation, and alkylation to produce the MMAB product. The hydroisomerization step is controlled by proper selection of the catalyst and operating conditions to favor the light branching required to selectively produce mono-methyl branch paraffins rather than paraffins with higher degrees of branching or branching in the wrong position. For feeds with inherent carbon chains longer than 9 - 14, the linear selective cracking step involved controlling the catalyst and operating condition to enhance the yield of chains in the 9 - 14 carbon number range.

Natural oils are not based on kerosene or other fossil fuels. Natural oils include those derived from plant or algal material, animal fats, nut and/or seed oils, and triglyceride-containing oils, and are often referred to as renewable oils. Natural oils typically comprise triglycerides, free fatty acids, or combinations thereof. Natural oils include, but are not limited to, Arachis oil (peanut oil; groundnut oil), Babassu oil, Coconut oil, Cottonseed oil, Grapeseed oil, Maize oil (corn oil), Mustard seed oil, Palm kernel oil, Palm oil, Palm olein (the liquid fraction derived from the fractionation of palm oil), Palm stearin (the high-melting fraction derived from the fractionation of palm oil), Rapeseed oil, Rapeseed oil - low erucic acid (low erucic acid turnip rape oil; low erucic acid colza oil; canola oil), Safflowerseed oil (safflower oil; 3arthamus oil; kurdee oil), Safflowerseed oil - high oleic acid (high oleic acid safflower oil; high oleic acid 3arthamus oil; high oleic acid kurdee oil), Sesameseed oil (sesame oil; gingelly oil; benne oil; ben oil; till oil; tillie oil) , Soya bean oil (soybean oil), Sunflowerseed oil (sunflower oil), and Sunflowerseed oil - high oleic acid (high oleic acid sunflower oil).

The methods for making mono-methyl alkylbenzenes from natural oils according to the present invention involve the deoxygenation of the natural oils to form paraffins. The C9 to C28 stream is sent to a separate linear selective cracking unit to crack the C14+ paraffins; the cracked paraffins are separated (by fractionation, distillations, and the like) into a first stream comprising the C9 to C14 normal and lightly branched paraffins, a second stream comprising C14+ (i.e., containing carbon chains from C15 to C28) paraffins, and a third stream comprising isoparaffins. The C9 to C14 paraffins from the linear selective cracking unit are isomerized to produce C9 to C14 mono-methyl paraffins. Contaminants, including but not limited to, comprise sulfur compounds, or nitrogen compounds, or phosphorous compounds, or oxygenates, or aromatics or combinations thereof, are removed from the isomerized C9 to C14 stream. The decontaminated stream is dehydrogenated to form olefins, di-olefins, and aromatics. The di-olefins are selectively hydrogenated to form additional olefins, and the aromatics are separated and removed forming an aromatics stream comprising the aromatics and a mono-olefin stream comprising the mono-olefins. Benzene is alkylated with the olefins, and the alkylation effluent comprises alkylbenzenes and benzene. The alkylbenzenes are then isolated.

The linear selective cracking and isomerization steps will be further described. The linear selective cracking takes place in a separate unit, rather than in the bottom bed of a first stage hydrocracking reactor because sulfur and nitrogen contaminants from the first stage can poison a metal-based hydrocracking catalyst. The C14+ paraffins are selectively cracked over the C9 to C14 due to higher absorption energy.

Selection of particular metal catalysts, including noble metals (such as ruthenium and platinum), and nickel can produce a much higher yield of normal paraffins with 9-14 carbons than previous processes. Suitable catalysts include, but are not limited to, Ru/ZrO₂, a Pt-Al₂O₃, Ni-alumina, or a NiOₓ/clay. With these catalysts, the C14+ stream is able to generate linear cracking products without significant amounts of branched isomer production.

Of the preferred catalysts, the Ru catalyst exhibits much higher activity and per-pass nC9 to nC14 yield than the other catalysts. Under the optimized reaction conditions, it also produces very small amounts of methane and isomerized product. This has been found to be the best catalyst for such chemical transformation process. The Pt-Al₂O₃ catalyst can produce even lower methane yield than the Ru based catalyst with slightly less linear product yield.

The linear selective cracking conditions comprise temperatures in a range of 290 °C to 455°C, or pressures in a range of 2.8 MPa to 17.5 MPa, or combinations thereof.

The first stream comprising C9 to C14 paraffins from the linear selective cracking unit is isomerized to produce C9 to C 14 mono-methyl paraffins. The weight ratio of mono-methyl paraffins to normal paraffins in the isomerized stream is in the range of 3 to 60.

The isomerization catalyst comprises a zeolite comprising a 10-ring AEL framework or combinations thereof. The isomerization conditions comprise a temperature in a range of 280 °C to 400°C, or a pressure in a range of 2.8 MPa to 17.5 MPa, or combinations thereof.

The overall process will now be described in more detail.

To limit catalyst deactivation, the feed is treated to remove sulfur contamination before hydrodeoxygenation. Otherwise, sulfur accumulates on the catalyst and leads to deactivation. A high temperature hydrogen treatment was shown to recover some of the lost activity. The degree of hydrodeoxygenation can affect the selectivity to each of the normal paraffins in the 9 to 14 carbon range. A large degree of hydrodeoxygenation can bias the hydrodeoxygenated composition largely in favor of normal dodecane and normal decane to the detriment of normal undecane and normal tridecane. A small degree of hydrodeoxygenation can bias the hydrodeoxygenated composition in favor of normal undecane and normal tridecane to the detriment of normal dodecane and normal decane.

The hydrodeoxygenation reactor temperatures are kept low, less than 343°C (650°F) for typical biorenewable feedstocks and less than 304°C (580°F) for feedstocks with higher free fatty acid (FFA) concentration to avoid polymerization of olefins found in FFA. Generally, hydrodeoxygenation reactor pressure of 700 kPa (100 psig) to 21 MPa (3000 psig) are suitable.

The linearity of the alkylbenzene product is mostly dependent on the linearity of the paraffins used to alkylate the benzene. It is a common rule of thumb by those skilled in the art that the linearity of a paraffin feed drops by 5-7 mass % after dehydrogenation and alkylation. Therefore, paraffin with 97 mass % linearity (or alternatively 3 mass % isoparaffin) would result in an alkylbenzene product with linearity around 90-92 mass %. This sets the requirement for paraffin linearity 5-7 mass % higher than the specification for the alkylbenzene product. Typically the linearity of the paraffin product is measured by UOP 621, UOP411, or UOP732 standard test method available from ASTM. Linear alkylbenzenes may be analyzed using ASTM Standard Test Method D4337.

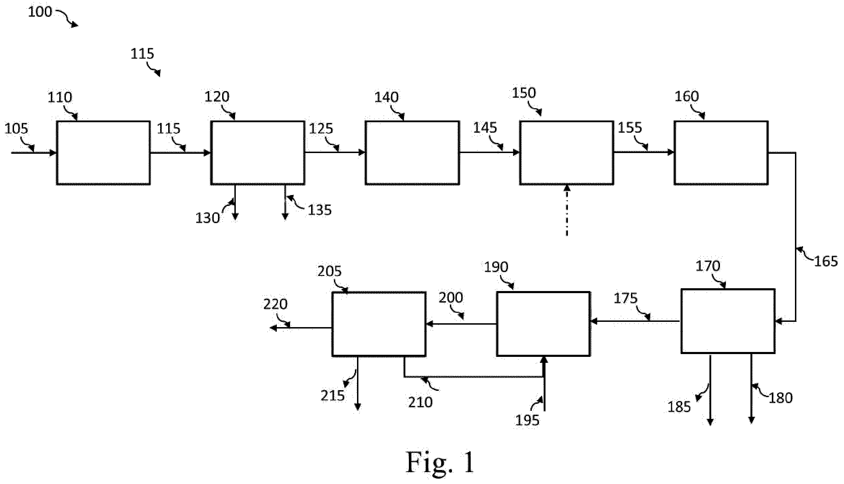

In Fig. 1, an exemplary system 100 for producing an alkylbenzene product from a specific triglyceride feed is illustrated.

In the illustrated embodiment, the selected natural oil feed 105 is delivered to a deoxygenation unit 110 which also receives a hydrogen feed (not shown). In the deoxygenation unit 110, the fatty acids in the natural oil feed 105 are deoxygenated and converted into normal paraffins. When the natural oil comprises triglycerides, the triglycerides are formed by three, typically different, fatty acid molecules that are bonded together with a glycerol bridge. The glycerol molecule includes three hydroxyl groups (HO--) and each fatty acid molecule has a carboxyl group (COOH). In triglycerides, the hydroxyl groups of the glycerol join the carboxyl groups of the fatty acids to form ester bonds. Therefore, during deoxygenation, the fatty acids are freed from the triglyceride structure and are converted into normal paraffins. The glycerol is converted into propane, and the oxygen in the hydroxyl and carboxyl groups is converted into water, carbon dioxide, or carbon monoxide. The deoxygenation reaction for fatty acids and triglycerides are respectively illustrated as:

H₂+ COOH → R + H₂O + CO₂

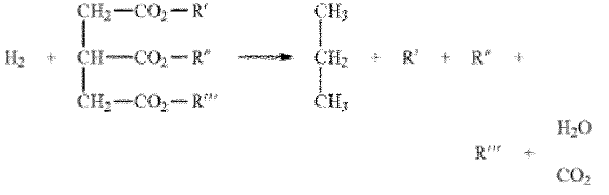

During the deoxygenation reaction, the length of a paraffin chain Rⁿ created will vary by a value of one depending on the exact reaction pathway. It is understood that deoxygenation includes at least one of hydrodeoxygenation, decarboxylation, and decarbonylation, or any combination thereof. For instance, if carbon dioxide is formed, then the chain will have one fewer carbon than the fatty acid source. If water is formed, then the chain will match the length of the fatty acid source.

Operating conditions for the deoxygenating unit include pressures in the range of from 250 to 800 psig (1724 to 5516 kPa) and temperatures in the range of from 274°C to 371 °C (525°F to 700°F) in one embodiment, from 274°C to 338°C (525°F to 640°F) in another embodiment and from 274°C to 310°C (525°F to 590°F) in another embodiment. Catalysts may include those containing one or more of Ni, Mo, Co, P, such as Ni--Mo, Ni--Mo--P, NiCo--Mo, or Co--Mo, on aluminas, silica, titania, zirconia, and mixtures thereof. Suitable hydrogen to hydrocarbon mole ratios include from 267.16 - 1781.08 m3/m3 (1500 to 10,000 scf/B), from 712.43 - 1602.97 m3/m3 (4000 to 9000 scf/B), and from 890.54 - 1424.8 m3/m3 (5000-8000 standard cubic feet per barrel of feedstock (scf/B)). Suitable space velocities include 0.2-3.0 hr⁻¹ LHSV. Conditions are selected to minimize cracking or isomerizing the paraffins.

The deoxygenated product contains normal paraffins, water, carbon dioxide, carbon monoxide, and propane.

The C9 to C28 stream 115 from the deoxygenation unit 110 is sent to the linear selective cracking unit 120 where it is selectively cracked to form a first stream 125 comprising normal or lightly branched C9 to C14 paraffins, a second stream 130 comprising C14+ paraffins 135, an a third stream comprising isoparaffins, as described above.

The first stream 125 is sent to the isomerization unit 140 where a portion of the C9 to C14 paraffins are converted to mono-methyl paraffins. The isomerization catalyst comprises a zeolite comprising a 10-ring AEL framework or combinations thereof. Suitable isomerization catalysts include, but are not limited to, SAPO- 11, AEI , AEL , AFO , AFX , ATO , BEA , CHA , FAU , FER , MEL, MFI, MOR , MRE , MTT , MWW or TON topology such as EU - 2 , ZSM - 11 , ZSM - 22 , ZSM - 23 , ZSM - 48 , SAPO - 5 , SAPO - 11 , SAPO - 31, SAPO - 34, SAPO - 41 , SSZ - 13 , SSZ - 16, SSZ - 39, MCM - 22, zeolite Y , ferrierite , mordenite , ZSM - 5 or zeolite beta, and combinations thereof.

The isomerization conditions comprise a temperature in a range of 280 °C to 400°C, or a pressure in a range of 2.8 MPa to 17.5 MPa, 267.16 - 1781.08 m³ of hydrogen per m³ of feed (1500-10000 standard cubic foot of hydrogen per barrel of feed), 0.25-2.5 LHSV or combinations thereof.

The overall process will now be described in more detail.

The isomerized stream 145 from the isomerization unit 140 is sent to a decontamination unit 150. The decontamination unit 150 removes contaminants in an adsorption system from the C9 to C14 mono-methyl paraffins in the isomerized stream 145. The contaminants include, but are not limited to, sulfur compounds, or nitrogen compounds, or phosphorous compounds, or oxygenates, or aromatics or combinations thereof.

The decontaminated stream 155 is sent to a dehydrogenation unit 160 where hydrogen is removed to produce a dehydrogenated stream 165 comprising mono-olefins, di-olefins, and aromatics. In the dehydrogenation unit 160, the paraffins are dehydrogenated into mono-olefins of the same carbon numbers as the paraffins. Typically, dehydrogenation occurs through known catalytic processes, such as the commercially popular Pacol process. Di-olefins (i.e., dienes) and aromatics are also produced as an undesired result of the dehydrogenation reactions as expressed in the following equations:
Mono-olefin formation: CₓH₂ₓ₊₂→ CₓH₂ₓ + H₂
Di-olefin formation: CₓH₂ₓ → CₓH₂ₓ₋₂ + H₂
Aromatic formation: CₓH₂ₓ₋₂ → CₓH₂ₓ₋₆ + 2H₂

Operating conditions for the dehydrogenation unit 160 include space velocities from 5 to 50 LHSV and from 20 to 32 LHSV; pressures from 34 kPa (g) to 345 kPa (g) (5 psig to 50 psig) and from 103 kPa (g) to 172 kPa (g) (15 psig to 25 psig); temperatures from 400°C to 500°C and from 440°C to 490°C, and hydrogen to hydrocarbon mole ratios from 1-12 and from 3-7. An example of a suitable catalyst is a Pt on alumina catalyst where platinum is attenuated with an attenuator metal. Another suitable catalyst is described in U.S. Pat. No. 6,177,381. The dehydrogenation unit 160 may be operated dry or with water injection up to 2000 mass-ppm water. Hydrogen can be recycled to the deoxygenation unit upstream.

The dehydrogenated stream 165 is sent to a selective hydrogenation unit 170, such as a DeFine reactor, where at least a portion of the di-olefins are hydrogenated to form additional mono-olefins. As a result, the mono-olefin stream 175 has an increased mono-olefin concentration compared to the dehydrogenated stream 165. The aromatics are separated and removed as aromatics stream 180. A light end stream 1185 containing any lights, such as butane, propane, ethane and methane, that resulted from cracking or other reactions during upstream processing can also removed.

The mono-olefin stream 175 comprising mono-olefins is sent to the alkylation unit 190 along with a benzene stream 195. The benzene is alkylated with the mono-olefins to form alkylbenzene. The alkylation unit 190 contains a catalyst, such as a solid acid catalyst, that supports alkylation of the benzene with the mono-olefins. Fluorinated silica-alumina, hydrogen fluoride (HF), aluminum chloride (AlCl₃), zeolitic, and ionic liquid catalysts are examples of major catalysts in commercial use for the alkylation of benzene with linear mono-olefins and may be used in the alkylation unit 190. As a result of alkylation, alkylbenzene, typically called linear alkylbenzene (LAB), is formed according to the reaction:

C₆H₆ + CₓH₂ₓ → C₆H₅CₓH₂ₓ₊₁

Suitable operating conditions for the alkylation unit 190 include space velocities from 1 to 10 LHSV, pressures to maintain liquid phase operation, such as 2068 kPa (g) to 4137 kPa (g) (300 psig to 600 psig), temperatures in the range of from 80°C to 180°C and 120°C to 170°C, benzene to olefin mole ratios of 3 to 40 and 8 to 35.

Surplus amounts of benzene are supplied to the alkylation unit 190 to achieve high degree of desired alkylation. Therefore, the alkylation effluent 200 exiting the alkylation unit 190 contains alkylbenzene and unreacted benzene. Further, the alkylation effluent 200 may also include some unreacted paraffins. The alkylation effluent 200 is passed to a benzene separation unit 205, such as a fractionation column, for separating the unreacted benzene and paraffins from the alkylation effluent 200. The unreacted benzene exits the benzene separation unit 205 in a benzene recycle stream 210 that may be sent back into the alkylation unit 190 to maintain the desired benzene/olefin ratio (e.g., 1-50) and to reduce the volume of fresh benzene needed. The fresh benzene requirement (i.e., the net benzene) is determined by the net olefin to the alkylation unit. A paraffin stream 215 can also be separated out and recycled to the dehydrogenation unit 160.

As a result of the post-alkylation separation processes, the linear alkylbenzene product 220 is isolated. It is noted that such separation processes are not necessary in all embodiments in order to isolate the linear alkylbenzene product 220.

The linear alkylbenzene product 220 is a linear alkylbenzene product comprising: alkylbenzenes having the formula C₆H₅CₙH₂ₙ₊₁ wherein n is from 9 to 14. In some embodiments, at least 80 mass % of the alkylbenzenes have linear alkyl groups, or at least 90 mass %.

The linear alkylbenzene may be sulfonated to provide a linear alkylbenzene sulfonate product comprising: alkylbenzene sulfonate compounds having the formula CᵤH₂ₙ₊₁CsHₐSO₃H wherein n is from 10 to 14, or wherein n is from 11 to 13.

In some embodiments, either before or after the decontamination step, the isomerized stream (if before the decontamination step) or the decontaminated stream (if after the decontamination step) can be separated in an absorption separation system (not shown) in which the mono-methyl paraffins are preferentially absorbed by the absorbent and the non-mono-methyl paraffins remain in the fluid phase. In some embodiments, the absorbent in the absorption separation system is divided into a plurality of absorbent beds.

The term "column" means a distillation column or columns for separating one or more components of different volatilities. Unless otherwise indicated, each column includes a condenser on an overhead of the column to condense and reflux a portion of an overhead stream back to the top of the column and a reboiler at a bottom of the column to vaporize and send a portion of a bottoms stream back to the bottom of the column. Feeds to the columns may be preheated. The top pressure is the pressure of the overhead vapor at the vapor outlet of the column. The bottom temperature is the liquid bottom outlet temperature. Unless indicated otherwise, overhead lines and bottoms lines refer to the net lines from the column downstream of any reflux or reboil take-off to the column. Stripper columns may omit a reboiler at a bottom of the column and instead provide heating requirements and separation impetus from a fluidized inert media such as steam.

As used herein, the term "a component-rich stream" or "a component stream" means that the stream coming out of a vessel has a greater concentration of the component than the feed to the vessel. As used herein, the term "a component-lean stream" means that the lean stream coming out of a vessel has a smaller concentration of the component than the feed to the vessel.

### EXAMPLES

### Example 1

A coconut oil feed was deoxygenated, to form paraffins, dehydrogenated to form mono-olefins, and benzene was alkylated with the mono-olefins to form an alkylbenzene product with a modern carbon content of 62 mass % modern carbon as determined by ASTM D6866 as compared to a theoretical modern carbon content of 66.4 mass %, a bromine number of 1 g Br/per gram sample as determined by UOP standard test method 304, and a linearity of 92 mass %.

### Example 2

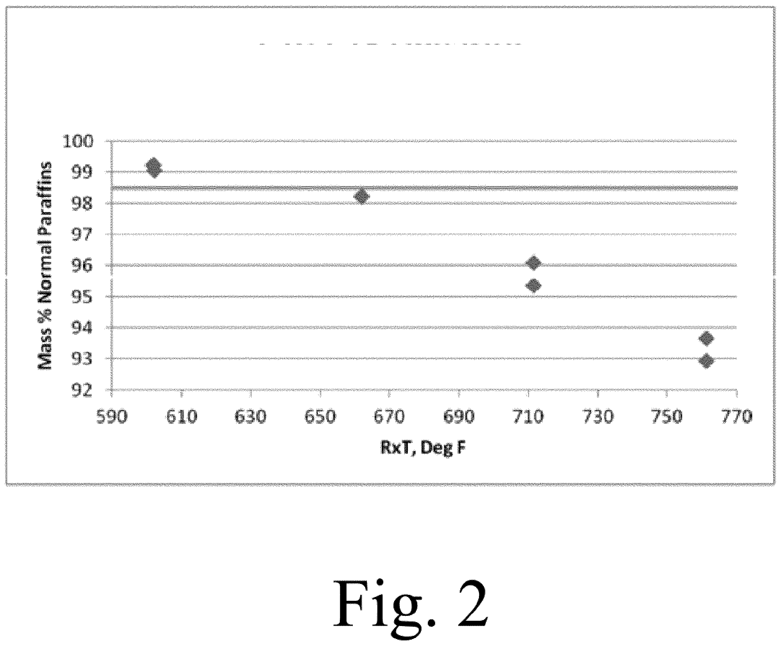

An oil was deoxygenated using a catalyst at a pressure of 3309 KPa (480 psig), H, to bio-oil ratio of 1285,38 m³/m³ (7200 scf/B) and a LHSV of 1 hr'. During operation, the deoxygenation reaction temperature was increased in steps from 315°C (600°F) to 349°C (660°F) and then to 377°C (710°F) and 404°C (760°F) to monitor the response of linearity in the final product to reaction temperature. The results are shown in Fig. 2 which is a plot of the concentration in mass % of normal C10-C 13 paraffins versus reaction temperature. Fig. 2 clearly demonstrates that as the deoxygenation reaction temperature is increased, the concentration of linear paraffins decreases. Controlling the temperature to less than 404°C (760°F) resulted in greater than 92 mass percent linear paraffins.
Note: Examples 1 and 2 were previously included in US Patent No. 9,079,814 as Examples 3 and 4.

In the foregoing, all temperatures are set forth in degrees Celsius and, all parts and percentages are by weight, unless otherwise indicated.

## Claims

1. A method for production of a mono methyl alkylbenzene product from a natural oil comprising:
deoxygenating the natural oil (105) to form a paraffin stream (115) comprising C9 to C28 carbon chains;
linear selective cracking the paraffin stream (115) in a separate linear selective cracking unit (120) under linear selective cracking conditions in the presence of a linear selective cracking catalyst to form a first stream (125) comprising normal or lightly branched C9 to C14 paraffins, a second stream (130) comprising C14+ paraffins, and a third stream (135) comprising iso paraffins;
isomerizing the first stream (125) under isomerization conditions in the presence of an isomerization catalyst to form an isomerized stream (145) comprising C9 to C14 mono-methyl paraffins, wherein the isomerization catalyst comprises a zeolite comprising a 10-ring AEL framework or combinations thereof;
removing contaminants from the isomerized stream (145) to form a decontaminated stream (155) wherein the contaminants comprise sulfur compounds, or nitrogen compounds, or phosphorous compounds, or oxygenates, or aromatics, or combinations thereof;
dehydrogenating the decontaminated stream (155) to provide a dehydrogenated stream (165) comprising mono-olefins, di-olefins, and aromatics;
selectively hydrogenating the di-olefins in the dehydrogenated stream (165) to form additional mono-olefins, and separating and removing the aromatics from the mono-olefins to form an aromatics stream (180) comprising the aromatics and a mono-olefins stream (175) comprising the mono-olefins:
alkylating benzene (195) with the mono-olefins under alkylation conditions to provide an alkylation effluent (200) comprising alkylbenzenes and benzene; and
isolating the alkylbenzenes to provide the alkylbenzene product (220) derived from the natural oil.

2. The method of claim 1 further comprising:
separating the isomerized stream (145) through a first absorption separation system, wherein the mono methyl paraffins in the mixture are preferentially adsorbed by a first adsorbent and where the non- mono methyl paraffins remain in the fluid phase, before removing the contaminants from the isomerized stream (145);
or
separating the decontaminated stream (155) through a second absorption separation system, wherein the mono methyl paraffins in the mixture are preferentially adsorbed by a second adsorbent and where the non- mono methyl paraffins remain in the fluid phase, before dehydrogenating the decontaminated stream (155).

3. The method of claim 2:
wherein the adsorbent in the first absorption separation system is divided into a plurality of adsorbent bed zones; or
wherein the adsorbent in the second absorption separation system is divided into a plurality of adsorbent bed zones;
or both.

4. The method of any one of claims 1-2 wherein the isomerized stream (145) comprises a weight ratio of mono-methyl paraffins to normal paraffins of 3 to 60.

5. The method of any one of claims 1-2 further comprising:
recycling the second stream (130) comprising C14+ paraffins to the linear selective cracking unit (120).

6. The method of any one of claims 1-2 wherein the linear selective cracking catalyst comprises a ruthenium, or a platinum, or a nickel supported catalyst, or mixtures thereof.

7. The method of any one of claims 1-2 wherein the linear selective cracking conditions comprise a temperature in a range of 290 °C to 455°C, or a pressure in a range of 2.8 MPa to 17.5 MPa, or combinations thereof.

8. The method of any one of claims 1-2 wherein the isomerization conditions comprise a temperature in a range of 280 °C to 400°C, or a pressure in a range of 2.8 MPa to 17.5 MPa, or 267.16 - 1781.08 m³ of hydrogen per m³ of feed (1500-10000 standard cubic foot of hydrogen per barrel of feed), or 0.25-2.5 LHSV, or combinations thereof.

9. The method of any one of claims 1-2 wherein the zeolite comprising the 10-ring AEL framework comprises SAPO-11.

10. The method of any one of claims 1-2 wherein the isomerization catalyst comprises platinum or nickel tungsten sulfide.

## Patentansprüche

1. Verfahren zur Herstellung eines Mono-Methylalkylbenzolprodukts aus einem natürlichen Öl, umfassend:
Desoxygenieren des natürlichen Öls (105), um einen Paraffinstrom (115) zu bilden, umfassend C9- bis C28-Kohlenstoffketten;
lineares selektives Cracken des Paraffinstroms (115) in einer separaten linear-selektiven Cracking-Einheit (120) unter linear-selektiven Cracking-Bedingungen in Anwesenheit eines linear-selektiven Cracking-Katalysators, um einen ersten Strom (125), umfassend normale oder leicht verzweigte C9- bis C14-Paraffine, einen zweiten Strom (130), umfassend C14+-Paraffine, und einen dritten Strom (135), umfassend Isoparaffine, zu bilden;
Isomerisieren des ersten Stroms (125) unter Isomerisierungsbedingungen in Anwesenheit eines Isomerisierungs-Katalysators, um einen isomerisierten Strom (145), umfassend C9- bis C14-Mono-Methylparaffine, zu bilden, wobei der Isomerisierungs-Katalysator einen Zeolith umfasst, der ein 10-Ring-AEL-Gerüst oder Kombinationen davon umfasst;
Entfernen von Kontaminationsstoffen aus dem isomerisierten Strom (145), um einen dekontaminierten Strom (155) zu bilden, wobei die Kontaminationsstoffe Schwefelverbindungen oder Stickstoffverbindungen oder Phosphorverbindungen oder Sauerstoffverbindungen oder Aromaten oder Kombinationen davon umfassen;
Dehydrieren des dekontaminierten Stroms (155), um einen dehydrierten Strom (165) bereitzustellen, der Mono-Olefine, Di-Olefine und Aromate umfasst;
selektives Hydrieren der Di-Olefine in dem dehydrierten Strom (165), um zusätzliche Mono-Olefine zu bilden, und Trennen und Entfernen der Aromaten aus den Mono-Olefinen, um einen Aromatenstrom (180) zu bilden, umfassend die Aromaten, und einen Mono-Olefinstrom (175), umfassend die Mono-Olefine:
Alkylieren von Benzol (195) mit den Mono-Olefinen unter Alkylierungsbedingungen, um einen Alkylierungsabstrom (200) bereitzustellen, umfassend Alkylbenzole und Benzol; und
Isolieren der Alkylbenzole, um das von dem natürlichen Öl abgeleitete Alkylbenzolprodukt (220) bereitzustellen.

2. Verfahren nach Anspruch 1, ferner umfassend:
Trennen des isomerisierten Stroms (145) durch ein erstes Absorptions-Trennsystem, wobei die Mono-Methylparaffine im Gemisch vorzugsweise von einem ersten Adsorbens adsorbiert werden und wobei die Nicht-Mono-Methylparaffine vor dem Entfernen der Kontaminationsstoffe aus dem isomerisierten Strom (145) in der flüssigen Phase verbleiben;
oder
Trennen des dekontaminierten Stroms (155) durch ein zweites Absorptions-Trennsystem, wobei die Mono-Methylparaffine in dem Gemisch vorzugsweise von einem zweiten Adsorbens adsorbiert werden und die Nicht-Mono-Methylparaffine vor dem Dehydrieren des dekontaminierten Stroms (155) in der flüssigen Phase verbleiben.

3. Verfahren nach Anspruch 2:
wobei das Adsorbens im ersten Absorptions-Trennsystem in eine Vielzahl von Adsorbensbettzonen unterteilt ist; oder
wobei das Adsorbens im zweiten Absorptions-Trennsystem in eine Vielzahl von Adsorbensbettzonen unterteilt ist;
oder beides.

4. Verfahren nach einem der Ansprüche 1 bis 2, wobei der isomerisierte Strom (145) ein Gewichtsverhältnis von Mono-Methylparaffinen zu Normalparaffinen von 3 bis 60 umfasst.

5. Verfahren nach einem der Ansprüche 1 bis 2, ferner umfassend:
Recyceln des zweiten Stroms (130), umfassend C14+-Paraffine, zu der linear-selektiven Cracking-Einheit (120).

6. Verfahren nach einem der Ansprüche 1 bis 2, wobei der linear-selektive Cracking-Katalysator einen Ruthenium-, oder einen Platin- oder einen Nickel-geträgerten Katalysator oder Gemische davon umfasst.

7. Verfahren nach einem der Ansprüche 1 bis 2, wobei die linear-selektiven Cracking-Bedingungen Folgendes umfassen: eine Temperatur in einem Bereich von 290 °C bis 455 °C oder einen Druck in einem Bereich von 2,8 MPa bis 17,5 MPa oder Kombinationen derselben.

8. Verfahren nach einem der Ansprüche 1 bis 2, wobei die Isomerisierungsbedingungen eine Temperatur im Bereich von 280 °C bis 400 °C oder einen Druck im Bereich von 2,8 MPa bis 17,5 MPa oder 267,16 bis 1781,08 m³ Wasserstoff pro m³ Zufuhr (1500 bis 10000 Standardkubikfuß Wasserstoff pro Barrel Zufuhr) oder 0,25 bis 2,5 LHSV oder Kombinationen davon umfassen.

9. Verfahren nach einem der Ansprüche 1 bis 2, wobei der Zeolith, der das 10-Ring-AEL-Gerüst umfasst, SAPO-11 umfasst.

10. Verfahren nach einem der Ansprüche 1 bis 2, wobei der Isomerisierungs-Katalysator Platin oder Nickel-Wolfram-Sulfid umfasst.

## Revendications

1. Procédé de production d'un produit monométhylalkylbenzène à partir d'une huile naturelle comprenant :
la désoxygénation de l'huile naturelle (105) pour former un flux de paraffine (115) comprenant des chaînes de carbone en C9 à C28 ;
le craquage sélectif linéaire du flux de paraffine (115) dans une unité distincte de craquage sélectif linéaire (120) dans des conditions de craquage sélectif linéaire en présence d'un catalyseur de craquage sélectif linéaire pour former un premier flux (125) comprenant des paraffines en C9 à C14 normales ou légèrement ramifiées, un deuxième flux (130) comprenant des paraffines en C14+ et un troisième flux (135) comprenant des isoparaffines ;
l'isomérisation du premier flux (125) dans des conditions d'isomérisation en présence d'un catalyseur d'isomérisation pour former un flux isomérisé (145) comprenant des paraffines monométhyliques en C9 à C14, dans lequel le catalyseur d'isomérisation comprend une zéolite comprenant une structure AEL à 10 anneaux ou des combinaisons de celle-ci ;
l'élimination des contaminants du flux isomérisé (145) pour former un flux décontaminé (155) dans lequel les contaminants comprennent des composés soufrés, ou des composés azotés, ou des composés phosphorés, ou oxygénés, ou aromatiques, ou des combinaisons de ceux-ci ;
la déshydrogénation du flux décontaminé (155) pour fournir un flux déshydrogéné (165) comprenant des mono-oléfines, des di-oléfines et des aromatiques ;
l'hydrogénation sélective des di-oléfines dans le flux déshydrogéné (165) pour former des mono-oléfines supplémentaires, et la séparation et l'élimination des aromatiques des mono-oléfines pour former un flux d'aromatiques (180) comprenant les aromatiques et un flux de mono-oléfines (175) comprenant les mono-oléfines ;
l'alkylation du benzène (195) avec les mono-oléfines dans des conditions d'alkylation pour fournir un effluent d'alkylation (200) comprenant des alkylbenzènes et du benzène ; et
l'isolation des alkylbenzènes pour fournir le produit alkylbenzène (220) dérivé de l'huile naturelle.

2. Procédé selon la revendication 1, comprenant en outre :
la séparation du flux isomérisé (145) à travers un premier système de séparation par absorption, dans lequel les paraffines monométhyliques dans le mélange sont adsorbées de préférence par un premier adsorbant et où les paraffines non monométhyliques restent dans la phase fluide, avant l'élimination des contaminants du flux isomérisé (145) ;
ou
la séparation du flux décontaminé (155) à travers un deuxième système de séparation par absorption, dans lequel les paraffines monométhyliques dans le mélange sont adsorbées de préférence par un deuxième adsorbant et où les paraffines non monométhyliques restent dans la phase fluide, avant la déshydrogénation du flux décontaminé (155).

3. Procédé selon la revendication 2 :
dans lequel l'adsorbant dans le premier système de séparation par absorption est divisé en une pluralité de zones de lit d'adsorbant ; ou
dans lequel l'adsorbant dans le deuxième système de séparation par absorption est divisé en une pluralité de zones de lit d'adsorbant ;
ou les deux.

4. Procédé selon l'une quelconque des revendications 1 à 2, dans lequel le flux isomérisé (145) comprend un rapport pondéral de paraffines mono-méthyliques sur des paraffines normales de 3 sur 60.

5. Procédé selon l'une quelconque des revendications 1 à 2, comprenant en outre :
le recyclage du deuxième flux (130) comprenant des paraffines en C14+ vers l'unité de craquage sélectif linéaire (120).

6. Procédé selon l'une quelconque des revendications 1 à 2, dans lequel le catalyseur de craquage sélectif linéaire comprend un catalyseur à base de ruthénium, de platine ou de nickel, ou des mélanges de ceux-ci.

7. Procédé selon l'une quelconque des revendications 1 à 2, dans lequel les conditions de craquage sélectif linéaire comprennent une température dans une plage de 290 °C à 455 °C, ou une pression dans une plage de 2,8 MPa à 17,5 MPa, ou des combinaisons de celles-ci.

8. Procédé selon l'une quelconque des revendications 1 à 2, dans lequel les conditions d'isomérisation comprennent une température dans une plage de 280 °C à 400 °C, ou une pression dans une plage de 2,8 MPa à 17,5 MPa, ou 267,16 à 1781,08 m³ d'hydrogène par m³ de charge (1500 à 10000 pieds cubes standard d'hydrogène par baril de charge), ou 0,25 à 2,5 LHSV, ou des combinaisons de ceux-ci.

9. Procédé selon l'une quelconque des revendications 1 à 2, dans lequel la zéolite comprenant la structure AEL à 10 anneaux comprend SAPO-11.

10. Procédé selon l'une quelconque des revendications 1 à 2, dans lequel le catalyseur d'isomérisation comprend du platine ou du sulfure de nickel-tungstène.
